# EUROPEAN PATENT SPECIFICATION

(11) **EP 4 412 686 B1**
(45) Date of publication and mention of the grant of the patent: **05.11.2025**
(21) Application number: 22786795.9
(22) Date of filing: 19.09.2022
(51) Int. Cl.: A61M 5/32

(54) **A CAP ASSEMBLY OF A MEDICAMENT DELIVERY DEVICE**
KAPPENANORDNUNG FÜR EINE MEDIKAMENTENABGABEVORRICHTUNG
ENSEMBLE CAPUCHON D'UN DISPOSITIF D'ADMINISTRATION DE MÉDICAMENTS

(30) Priority: 04.10.2021 EP 21200830
(43) Date of publication of application: 14.08.2024
(73) Proprietor: SHL Medical AG, 6302 Zug (CH)
(72) Inventor: OLSON, Stephan, 131 28 Nacka Strand (SE)
(86) International application number: PCT/EP2022/075902
(87) International publication number: WO 2023/057194

(56) References cited:
- WO-A1-2017/089267
- WO-A1-2017/089281
- WO-A1-2020/074570
- US-A1- 2020 254 189

## Description

### TECHNICAL FIELD

The present disclosure generally relates to a cap assembly of a medicament delivery device, and particularly to a cap assembly comprising a rotatable connector.

### BACKGROUND

There are many medicament delivery devices on the market that have been developed for self-administration of medicament, where one large group is medicament injection devices. Many of these devices have been provided with removable delivery member shields to fully cover a medicament delivery member, e.g. a needle or a nozzle, to keep the medicament delivery member sterile. For removing the delivery member shield, the medicament delivery device usually comprises a cap assembly. The cap assembly comprises a shield remover tightly gripping on the medicament delivery member shield so that when the cap assembly has been removed, the medicament delivery member shield can also be removed. However, usually, a friction between the medicament delivery member and the medicament delivery member shield is significant, and thus the cap assembly is difficult to be removed for some users, e.g. elders or people with impaired hands, to remove the cap assembly. Documents WO 2020/074570 A1, US 2020/254189 A1, WO 2017/089267 A1 and WO 2017/089281 A1 disclose prior art cap assemblies.

It has been appreciated that solutions for reducing the required force for the cap assembly with the shield remover; thus, an easy to be used medicament delivery device for a wide range of users would be beneficial.

### SUMMARY

The invention is defined by the appended claims, to which reference should now be made.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which during use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which during use of the medicament delivery device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", or "circumferentially" refer to a circumference or a circumferential direction relative to an axis, typically a central axis extending in the direction of the longest extension of the device and/or component. Similarly, "radial" or "radially" refer to a direction extending radially relative to the axis, and "rotation", "rotational" and "rotationally" refer to rotation relative to the axis.

There is hence provided with cap assembly of a medicament delivery device, the cap assembly comprising: a tubular outer shell, a shield remover, and a connector; the tubular outer shell extends along a longitudinal axis between a proximal end and a distal end; the shield remover is at least partially arranged within the tubular outer shell; the shield remover is movable relative to the tubular outer shell in the direction of the longitudinal axis; and the connector is arranged within the tubular outer shell; the connector is movably connected to both the tubular outer shell and the shield remover, the connector is configured to be moved in the proximal direction by a movement of the tubular outer shell in the proximal direction; such that the shield remover is moved a distance smaller than a distance moved by the tubular outer shell.

A user is configured to grip on the tubular outer shell and remove the cap assembly by pulling the tubular outer shell relative to a housing of the medicament delivery device. The shield remover is configured to grip on a medicament delivery member shield, and the shield remover is movably connected to the tubular outer shell via the connector. Thus, the connector causes a different length of a moving distance between the tubular outer shell and the shield remover. When the tubular outer shell is pulled by the user, the tubular outer shell will move a longer distance than the shield remover in the longitudinal axis; therefore, the user can remove the shield remover with a lower force in comparison with a cap assembly that the tubular outer shell is axially fixed to the shield remover.

Preferably, according to another embodiment, the connector is rotatably attached to either the tubular outer shell or the shield remover, so that a movement of the tubular outer shell relative to the shield remover in the direction of the longitudinal axis causes the connector to rotate relative to the tubular outer shell.

Preferably, according to another embodiment, the connector is rotatably attached to the tubular outer shell; and the connector is pivotally attached to the tubular outer shell.

Preferably, according to another embodiment, the connector comprises a connector body; and the connector body comprises a ring or a circlip.

Preferably, according to another embodiment, the cap assembly comprises an axial extension extending in the direction of the longitudinal axis; and the axial extension is arranged between the shield remover and the connector body.

Preferably, according to another embodiment, the connector body comprises the axial extension extending in the direction of the longitudinal axis from the connector body towards the shield remover.

Preferably, according to another embodiment, the axial extension of the connector body is adjacent to a distally directed surface of the shield remover.

Alternatively, according to another embodiment, the shield remover comprises the axial extension extending in the direction of the longitudinal axis towards the connector body.

Preferably, according to another embodiment, the connector body comprises a transverse extension extending from the connector body in a direction transverse to the longitudinal axis; and the transverse extension is pivotally attached to the tubular outer shell.

Preferably, according to another embodiment, a cut-out or recess is arranged in a wall of the tubular outer shell; and the transverse protrusion of the connector is positioned within the cut-out or recess.

Alternatively, according to another embodiment, the connector comprises a rotator rotatable relative to both the tubular outer shell and the shield remover and axially movable relative to the tubular outer shell; the rotator is rotatable around an axis perpendicular to a plane parallel to the longitudinal axis; and the rotator is adjacent to the tubular outer shell.

Preferably, according to another embodiment, the connector is rotatably attached to the shield remover, and wherein the connector is a wheel.

Preferably, according to another embodiment, the connector is rotatably attached to the shield remover, the shield remover comprises an arm extending in the distal direction relative to the tubular outer shell; and the rotator is rotatably attached to the arm of the shield remover.

Preferably, according to another embodiment, the rotator is configured to engage an outer surface of a housing of the medicament delivery device.

Alternatively, according to another embodiment, the rotator is configured to engage an outer surface of a delivery guard of the medicament delivery device.

Preferably, according to another embodiment, the rotator comprises a rubber surface or a rough surface.

Preferably, according to another embodiment, the connector comprises two rotators arranged opposite to one another relative to the longitudinal axis.

Preferably, according to another embodiment, the rotator can be a wheel, a rod, or a ball or a ball bearing set.

Preferably, according to another embodiment, the shield remover is movably attached to the tubular outer shell of the cap assembly by a snap-fit engagement.

Preferably, according to another embodiment, the snap-fit engagement is formed by a snap-fit groove and a snap-fit arm; and the snap-fit arm is movable within the snap-fit groove in a predetermined distance.

Preferably, according to another embodiment, the shield remover comprises an inner body, the inner body comprising a gripper for engaging a delivery member shield of the medicament delivery device; and the gripper extends from a wall of the inner body.

Preferably, according to another embodiment, the inner body extends along the longitudinal axis between a proximal end and a distal end; and wherein the gripper is arranged closer to the distal end of the inner body than to the proximal end of the inner body.

Another aspect of the invention provides the sub-assembly further comprises a housing for receiving a medicament container of the medicament delivery device; the housing extends along the longitudinal axis between a proximal end and a distal end; the cap assembly is removably attached to the proximal end of the housing; the housing comprises a proximally directed surface at the proximal end of the housing; the proximally directed surface inclines from a first side to a second side relative to a plane perpendicular to the longitudinal axis; the connector is adjacent to the proximally directed surface; and the connector is pivotally attached to the tubular outer shell at a point next to the first end of the proximally directed surface.

Another aspect of the invention provides a medicament delivery device comprising a cap assembly or a sub-assembly as described above.

Preferably, according to another embodiment, the medicament delivery device can be an injector, an inhaler, or a medical sprayer.

Preferably, according to another embodiment, the injector is an autoinjector, an insulin pen, and a safety syringe.

Preferably, according to another embodiment, the needle guard of the injector can be a user-detachable needle guard or a non-detachable needle guard for an end-user.

Generally, all terms used in the claims are to be interpreted according to their ordinary meaning in the technical field, unless explicitly defined otherwise herein. All references to "a/an/the element, apparatus, component, means, etc." are to be interpreted openly as referring to at least one instance of the element, apparatus, component, means, etc., unless explicitly stated otherwise.

### BRIEF DESCRIPTION OF THE DRAWINGS

Embodiments of the inventive concept will now be described, by way of example only, with reference to the accompanying drawings, in which:
Fig. 1 schematically shows a perspective view of a cap assembly.
Fig. 2 schematically shows a side view of a shield remover of the cap assembly of Fig. 1 with an inner body.
Fig. 3 schematically shows a perspective view of a housing of a sub-assembly comprising the cap assembly of Fig. 1.
Fig. 4 schematically shows a perspective view of the cap assembly of Fig. 1.
Fig. 5 schematically shows a perspective view of the cap assembly of Fig. 1 arranged with the housing of Fig. 3.
Fig. 6 schematically shows a cross-section view of the inner body of Fig. 2 arranged on a medicament delivery device.
Figs 7A-7C schematically show cross-section views of the cap assembly of Fig. 1 during removing the cap assembly from the housing of Fig. 3.
Fig. 8 schematically shows a perspective view of the cap assembly in another example.
Fig. 9 schematically shows a cross-section view of the cap assembly of Fig. 8.
Fig. 10 schematically shows a cross-section view of the cap assembly of Fig. 8 with the inner body of Fig. 2.
Figs 11A-11B schematically show cross-section views of the cap assembly of Fig. 8 during removing the cap assembly from a housing of a medicament delivery device.

### DETAILED DESCRIPTION

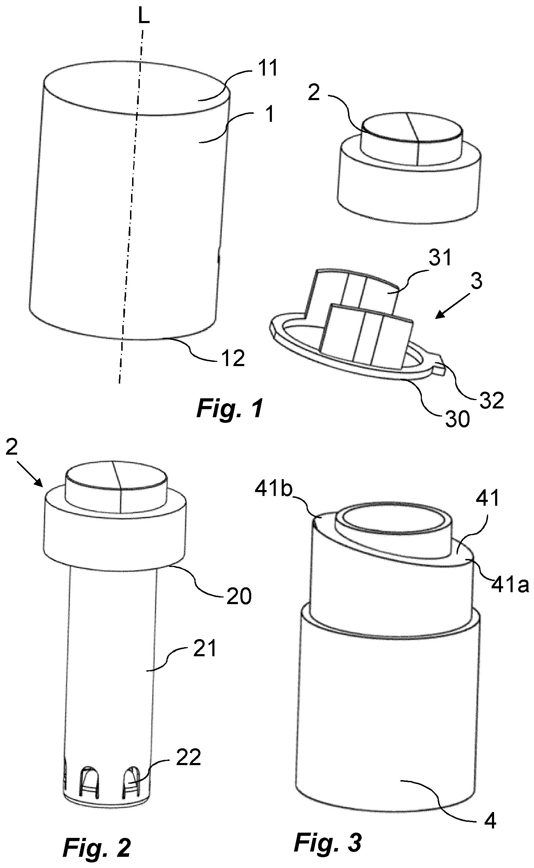

Figs 1-11B illustrate a cap assembly of a medicament delivery device. The cap assembly comprises a tubular outer shell 1, a shield remover 2; 2', and a connector 3; 3'. The tubular outer shell 1 extends along a longitudinal axis L between a proximal end 11 and a distal end 12. The shield remover 2 is at least partially arranged within the tubular outer shell 1. The shield remover 2 is movable relative to the tubular outer shell 1 in the direction of the longitudinal axis L. The connector 3; 3' is arranged within the tubular outer shell 1. The connector 3; 3' is movably connected to both the tubular outer shell 1 and the shield remover 2. The connector 3; 3' is configured to be moved in the proximal direction by a movement of the tubular outer shell 1 in the proximal direction, such that the shield remover 2 is moved a distance smaller than a distance moved by the tubular outer shell 1.

Optionally, the shield remover 2; 2' is movably attached to the tubular outer shell 1 of the cap assembly. In one example, the shield remover 2; 2' is movably attached to the tubular outer shell 1 of the cap assembly by a snap-fit engagement. In a preferred example, the snap-fit engagement is formed by a snap-fit groove and a snap-fit arm; and the snap-fit arm is movable within the snap-fit groove at a predetermined distance.

In one example, the proximal end of the tubular outer shell is a sealed end. In another example, the proximal end of the tubular outer shell comprises one or more apertures. The tubular outer shell can be formed in different shapes, e.g. cylindrical, triangle, rectangular, or oval (observing along the longitudinal axis L) dependent on the design of the medicament delivery device that comprises the cap assembly of the invention. In one example, the tubular outer shell comprises a gripping facilitating part, such as on an outer surface of the tubular outer shell over-molding or coating with a material that can increase friction when the user grips on the outer surface of the tubular outer shell; or the gripping facilitating part can be a series ridges and grooves arranged on the outer surface of the tubular outer shell.

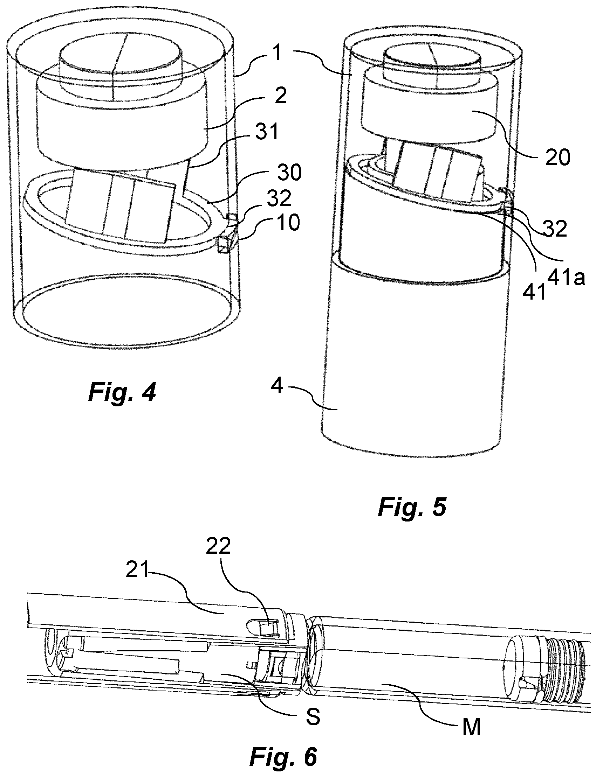

The shield remover 2; 2' is at least partially arranged within the tubular outer shell 1 and the shield remover 2; 2' is movable relative to the tubular outer shell 1 in the direction of the longitudinal axis L. The shield remover 2; 2' is configured to grip on a medicament delivery member shield, e.g. a rigid needle shield or a resilient delivery member sheath. The shield remover 2; 2' can be formed in any suitable shape, such as tubular-shaped or one or more pairs of arms. The shield remover 2; 2' comprises one or more grippers configured to engage with the medicament delivery member shield. For example, the one or more grippers can be one or more spikes penetrating into the resilient delivery member sheath or one or more ledge abutting on an edge of the rigid needle shield or the resilient delivery member sheath. The shield remover can be a single component or can be formed by multiple components attached to one another. In one example, the shield remover 2; 2' comprises an inner body 21 comprising the gripper 22 for engaging with a delivery member shield S of the medicament delivery device M, as shown in Fig. 6. The gripper is arranged in a wall of the inner body 21. In one example, the inner body 21 extends along the longitudinal axis L between a proximal end and a distal end. The gripper 22 is arranged closer to the distal end than the proximal end of the inner body 21. Alternatively, the gripper can be arranged closer to the proximal end than the distal end of the inner body. In a preferred example, the gripper 22 is an arm comprising a proximally directed surface. For example, a cut-out is arranged in the wall of the inner body, the arm extends from a distal end of the cut-out towards the proximal end of the inner body, and the proximally directed surface is defined by a proximal end of the arm. In another example the arm extends from the wall of the inner body in the distal direction relative to the shield remover 2; 2', the arm comprises a hook at its distal end.

The connector 3; 3' is rotatably attached to either the tubular outer shell 1 or the shield remover 2; 2', so that a movement of the tubular outer shell 1 relative to the shield remover 2; 2' in the direction of the longitudinal axis L causes the connector 3; 3' to rotate relative to the tubular outer shell 1.

The connector 3; 3' causes the tubular outer shell moves a longer distance than the shield remover in the longitudinal axis L; therefore, a user can remove the shield remover with a lower force in comparison with a cap assembly that the tubular outer shell is axially fixed to the shield remover.

In one example, the connector 3 is rotatably attached to the tubular outer shell 1 as shown in Fig. 1 and Fig. 4. In a preferred example, the connector is pivotally attached to the tubular outer shell 1. Preferably, the connector 3 comprises a connector body 30 pivotally attached to the tubular outer shell 1. In one example, the connector body comprises a ring or circlip or an oval-shaped or rectangular-shaped section (observing along the longitudinal axis) extending in a direction transverse to the longitudinal axis L between a first end that is not attached to the tubular outer shell and a second end that is attached to the tubular outer shell. In another example, the connector 3 comprises a transverse extension 32 extending from the connector body 30 in the direction transverse to the longitudinal axis L. The transverse extension 32 is pivotally attached to the tubular outer shell 1. In one example, a cut-out or recess 10 is arranged in a wall of the tubular outer shell 1. The transverse extension 32 of the connector 3 is positioned within the cut-out or recess 10.

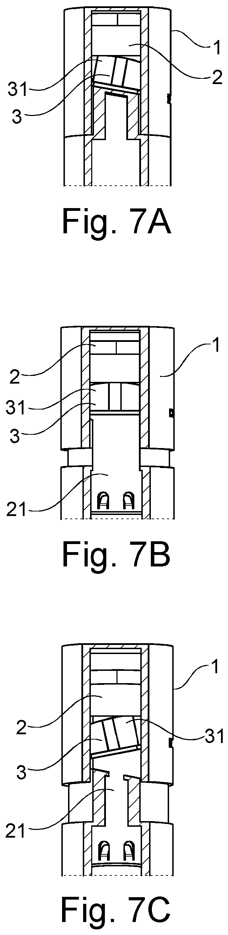

In a preferable example, the cap assembly of the invention comprises an axial extension extending in the direction of the longitudinal axis L. The axial extension is arranged between the shield remover and the connector body. The axial extension is configured to transfer the force from the tubular outer shell 1 to the shield remover 2 when the connector pivots relative to the tubular outer shell 1. In a preferred example, the connector 3 comprises the axial extension 31. The axial extension 31 extends in the direction of the longitudinal axis L from the connector body 30. The axial extension 31 of the connector 3 is adjacent to a distally directed surface 20 of the shield remover 2. Therefore, when the tubular outer shell moves in direction of the longitudinal axis L, the connector body 30 pivots relative to the tubular outer shell 1. The axial extension 31 pushes the distally directed surface 20 of the shield remover 2 so that the shield remover moves in the direction of the longitudinal axis L, as shown in Figs 7A-7C. Alternatively, the shield remover comprises the axial extension extending in the direction of the longitudinal axis towards the connector body. In the latter example, the axial extension is adjacent to the connector body when the connector pivots relative to the tubular outer shell.

In one example where the connector 3 comprises the transverse extension 32 positioned within the cut-out or recess 10 of the tubular outer shell 1, the transverse extension 32 can extend further away from the outer surface of the tubular outer shell. In this example, before gripping on the tubular outer shell, the user can press on the transverse extension to overcome the static friction between the medicament delivery member shield and a medicament delivery member of the medicament delivery device. In this example, the connector 3 acts as a lever.

Furthermore, the cap assembly can be a part of a sub-assembly of a medicament delivery device. The sub-assembly comprises a housing 4 for receiving a medicament container of the medicament delivery device. The housing 4 extends along the longitudinal axis L between a proximal end and a distal end. The cap assembly is removably attached to the proximal end of the housing 4. The housing 4 comprises a proximally directed surface 41 at the proximal end of the housing 4, as shown in Fig, 3. The proximally directed surface 41 inclines from a first side 41a to the second side 41b relative to a plane perpendicular to the longitudinal axis L. The connector 3 is adjacent to the proximally directed surface 41, as shown in Fig. 5. The connector 3 is pivotally attached to the tubular outer shell 1 at a point next to the first end 41a of the proximally directed surface 41, as shown in Fig. 5. In this example, the axial extension of the connector 3 is in contact with the distally directed surface 20 of the shield remover 2 so that more force applied on the tubular outer shell can be transferred to the shield remover 2.

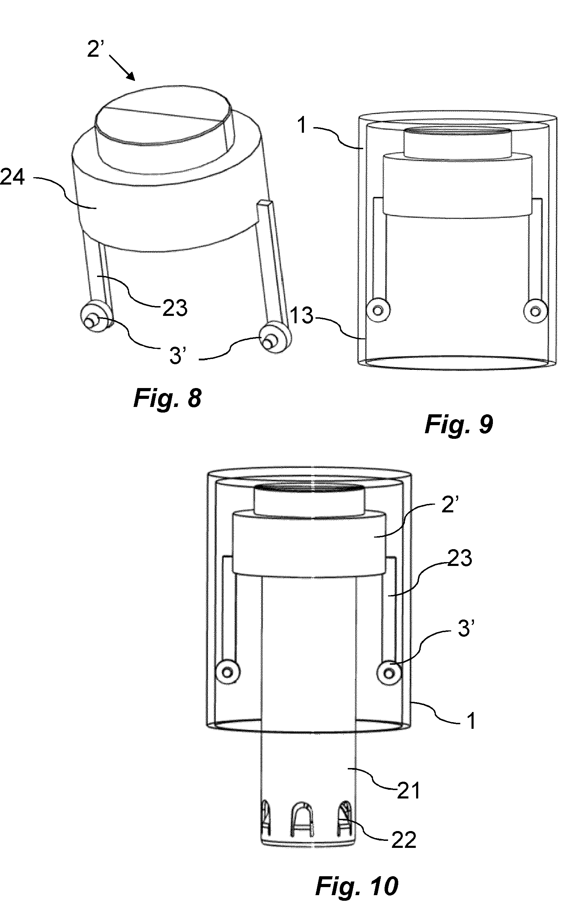

In another example, as shown in Figs 8-10, the connector 3' is rotatably attached to the shield remover 2'. In this example, the connector 3' comprises a rotator 3' rotatable relative to both the tubular outer shell 1 and the shield remover 2' and axially movable relative to the tubular outer shell 1. The rotator 3' is rotatable around an axis perpendicular to a plane parallel to the longitudinal axis L. The rotator is adjacent to the tubular outer shell 1. The rotator 3' is axially immovable relative to the shield remover 2'.

The rotator 3' is configured to engage an outer surface of a housing of the medicament delivery device or an outer surface of a delivery guard of the medicament delivery device. Therefore, when the tubular outer shell 1 is pulled (i.e. moved in the proximal direction relative to the shield remover 2'), the friction between the rotator 3' and the tubular outer shell 1 causes the rotator 3' to rotate. When the rotator rotates, the friction between the rotator and either the outer surface of the housing of the medicament delivery device or the outer surface of the delivery guard of the medicament delivery device causes the rotator 3' to move the shield remover 2' in the proximal direction relative to the tubular outer shell 1.

In one preferred example, the shied remover 2' comprises a remover body 24 and an arm 23 extending from an outer surface of the remover body 24 in the distal direction of the tubular outer shell 1. The connector 3' is rotatably attached to the arm 23 of the shield remover 2'. The connector 3' is adjacent to the tubular outer shell 1 of the cap assembly. In one example, the connector 3' comprises a wheel rotatably attached to the arm 23 of the shield remover 2'. The wheel 3' is rotatable relative to the arm 23 of the shield remover 2' on a plane parallel to the longitudinal axis L. The wheel 3' is adjacent to an inner surface of the tubular outer shell 1 of the cap assembly. In one example, the wheel 3' is configured to engage an outer surface 40' of a housing 4' of the medicament delivery device when the cap assembly is attached to the medicament delivery device. Alternatively, in another example, the wheel is configured to engage an outer surface of a delivery guard of the medicament delivery device when the cap assembly is attached to the medicament delivery device.

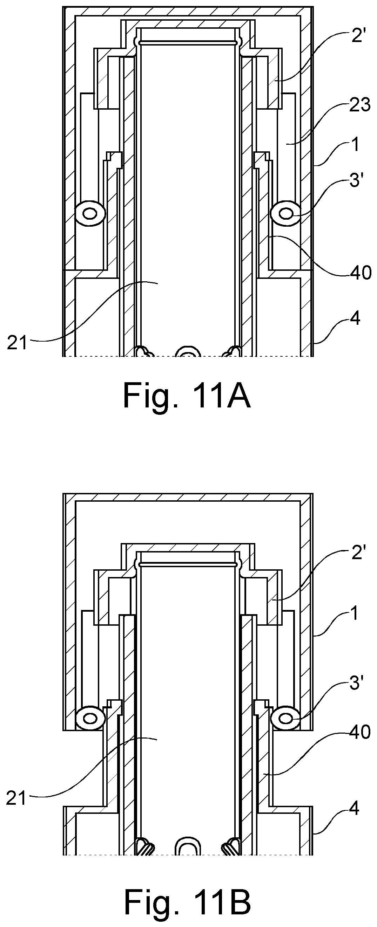

In this example, friction between the inner surface of the tubular outer shell 1 and the wheel 3' together with friction between the outer surface of either the housing 4' or the delivery guard and the wheel 3' causes the wheel 3' to move in the direction of the longitudinal axis L when the tubular outer shell 1 moves in the direction of the longitudinal axis L, as shown in Figs 11A-11B. In one example, the connector 3' comprises one or more pairs of wheels.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A cap assembly of a medicament delivery device, the cap assembly comprising:
a tubular outer shell (1), a shield remover (2), and a connector (3);
wherein the tubular outer shell (1) extends along a longitudinal axis (L) between a proximal end (11) and a distal end (12);
wherein the shield remover (2) is at least partially arranged within the tubular outer shell (1); wherein the shield remover (2) is movable relative to the tubular outer shell (1) in the direction of the longitudinal axis (L); and
wherein the connector (3) is arranged within the tubular outer shell (1);
wherein the connector (3) is movably connected to both the tubular outer shell (1) and the shield remover (2), wherein the connector (3) is configured to be moved in the proximal direction by a movement of the tubular outer shell in the proximal direction; such that the shield remover (2) is moved a distance smaller than a distance moved by the tubular outer shell (1);
wherein the connector (3) is rotatably attached to the tubular outer shell (1); and **characterized in that** the connector (3) is pivotally attached to the tubular outer shell;
and wherein the connector comprises a connector body; and wherein the connector body comprises a ring or a circlip.

2. The cap assembly according to claim 1, wherein the connector (3) is rotatably attached to either the tubular outer shell (1) or the shield remover (2), so that a movement of the tubular outer shell (1) relative to the shield remover (2) in the direction of the longitudinal axis (L) causes the connector (3) to rotate relative to the tubular outer shell (1).

3. The cap assembly according to claim 1 or 2, wherein the cap assembly comprises an axial extension extending in the direction of the longitudinal axis; and wherein the axial extension is arranged between the shield remover and the connector body.

4. The cap assembly according to claim 3, wherein the connector body comprises the axial extension extending in the direction of the longitudinal axis from the connector body towards the shield remover.

5. The cap assembly according to claim 4, wherein the axial extension of the connector body is adjacent to a distally directed surface of the shield remover.

6. The cap assembly according to any one of preceding claims, wherein the connector body comprises a transverse extension extending from the connector body in a direction transverse to the longitudinal axis; and wherein the transverse extension is pivotally attached to the tubular outer shell.

7. The cap assembly according to claim 6, wherein a cut-out or recess is arranged in a wall of the tubular outer shell; and wherein the transverse protrusion of the connector is positioned within the cut-out or recess.

8. The cap assembly according to any one of the preceding claims, wherein the shield remover comprises an inner body, the inner body comprising a gripper for engaging a delivery member shield of the medicament delivery device; and wherein the gripper extends from a wall of the inner body.

9. The cap assembly according to claim 8, wherein the inner body extends along the longitudinal axis between a proximal end and a distal end; and wherein the gripper is arranged closer to the distal end of the inner body than to the proximal end of the inner body.

10. The cap assembly according to any one of the preceding claims, wherein the shield remover is movably attached to the tubular outer shell of the cap assembly by a snap-fit engagement.

11. The cap assembly according to claim 10, wherein the snap-fit engagement is formed by a snap-fit groove and a snap-fit arm; and wherein the snap-fit arm is movable within the snap-fit groove in a predetermined distance.

12. A sub-assembly of a medicament delivery device, the sub-assembly comprising the cap assembly according to any one of claim 3-11, wherein the sub-assembly comprises a housing for receiving a medicament container of the medicament delivery device; wherein the housing extends along the longitudinal axis between a proximal end and a distal end; wherein the cap assembly is removably attached to the proximal end of the housing; wherein the housing comprises a proximally directed surface at the proximal end of the housing; wherein the proximally directed surface inclines from a first side to a second side relative to a plane perpendicular to the longitudinal axis; wherein the connector is adjacent to the proximally directed surface; and wherein the connector is pivotally attached to the tubular outer shell at a point next to the first end of the proximally directed surface.

## Patentansprüche

1. Kappenanordnung einer Medikamentenabgabevorrichtung, die Kappenanordnung umfassend:
eine röhrenförmige Außenhülle (1), einen Schutzentferner (2) und einen Verbinder (3);
wobei die röhrenförmige Außenhülle (1) sich entlang einer Längsachse (L) zwischen einem proximalen Ende (11) und einen distalen Ende (12) erstreckt;
wobei der Schutzentferner (2) mindestens teilweise innerhalb der röhrenförmigen Außenhülle (1) angeordnet ist; wobei der Schutzentferner (2) relativ zu der röhrenförmigen Außenhülle (1) in der Richtung der Längsachse (L) bewegbar ist; und
wobei der Verbinder (3) innerhalb der röhrenförmigen Außenhülle (1) angeordnet ist;
wobei der Verbinder (3) sowohl mit der röhrenförmigen Außenhülle (1) als auch mit dem Schutzentferner (2) bewegbar verbunden ist, wobei der Verbinder (3) konfiguriert ist, um durch eine Bewegung der röhrenförmigen Außenhülle in der proximalen Richtung in die proximale Richtung bewegt zu werden; derart, dass der Schutzentferner (2) um eine Distanz bewegt wird, die kleiner als eine Distanz ist, die durch die röhrenförmige Außenhülle (1) bewegt wird;
wobei der Verbinder (3) an der röhrenförmigen Außenhülle (1) drehbar befestigt ist;
und **dadurch gekennzeichnet, dass**
der Verbinder (3) an der röhrenförmigen Außenhülle schwenkbar befestigt ist;
und wobei der Verbinder einen Verbinderkörper umfasst; und wobei der Verbinderkörper einen Ring oder einen Sicherungsring umfasst.

2. Kappenanordnung nach Anspruch 1, wobei der Verbinder (3) entweder an der röhrenförmigen Außenhülle (1) oder dem Schutzentferner (2) drehbar befestigt ist, so dass eine Bewegung der röhrenförmigen Außenhülle (1) relativ zu dem Schutzentferner (2) in der Richtung der Längsachse (L) den Verbinder (3) veranlasst, sich relativ zu der röhrenförmigen Außenhülle (1) zu drehen.

3. Kappenanordnung nach Anspruch 1 oder 2, wobei die Kappenanordnung eine axiale Erstreckung umfasst, die sich in der Richtung der Längsachse erstreckt; und wobei die axiale Erstreckung zwischen dem Schutzentferner und dem Verbinderkörper angeordnet ist.

4. Kappenanordnung nach Anspruch 3, wobei der Verbinderkörper die axiale Erstreckung umfasst, die sich in der Richtung der Längsachse von dem Verbinderkörper zu dem Schutzentferner erstreckt.

5. Kappenanordnung nach Anspruch 4, wobei die axiale Erstreckung des Verbinderkörpers an eine distal gerichtete Oberfläche des Schutzentferners angrenzt.

6. Kappenanordnung nach einem der vorstehenden Ansprüche, wobei der Verbinderkörper eine Quererstreckung umfasst, die sich von dem Verbinderkörper in einer Richtung quer zu der Längsachse erstreckt; und wobei die Quererstreckung an der röhrenförmigen Außenhülle schwenkbar befestigt ist.

7. Kappenanordnung nach Anspruch 6, wobei ein Ausschnitt oder eine Aussparung in einer Wand der röhrenförmigen Außenhülle angeordnet ist; und wobei der Quervorsprung des Verbinders innerhalb des Ausschnitts oder der Aussparung positioniert ist.

8. Kappenanordnung nach einem der vorstehenden Ansprüche, wobei der Schutzentferner einen inneren Körper umfasst, der innere Körper umfassend einen Greifer zum Ineingriffnehmen eines Abgabeelementschutzes der Medikamentenabgabevorrichtung; und wobei sich der Greifer von einer Wand des inneren Körpers erstreckt.

9. Kappenanordnung nach Anspruch 8, wobei sich der innere Körper entlang der Längsachse zwischen einem proximalen Ende und einem distalen Ende erstreckt; und wobei der Greifer näher an dem distalen Ende des inneren Körpers als an dem proximalen Ende des inneren Körpers angeordnet ist .

10. Kappenanordnung nach einem der vorstehenden Ansprüche, wobei der Schutzentferner durch einen schnappverschließbaren Eingriff an der röhrenförmigen Außenhülle der Kappenanordnung bewegbar befestigt ist.

11. Kappenanordnung nach Anspruch 10, wobei der schnappverschließbare Eingriff durch eine schnappverschließbaren Nut und einen schnappverschließbaren Arm ausgebildet wird; und wobei der schnappverschließbare Arm innerhalb der schnappverschließbaren Nut über eine zuvor bestimmte Distanz bewegbar ist.

12. Unteranordnung einer Medikamentenabgabevorrichtung, die Unteranordnung umfassend die Kappenanordnung nach einem der Ansprüche 3 bis 11, wobei die Unteranordnung ein Gehäuse zum Aufnehmen eines Medikamentenbehälters der Medikamentenabgabevorrichtung umfasst; wobei das Gehäuse sich entlang der Längsachse zwischen einem proximalen Ende und einem distalen Ende erstreckt; wobei die Kappenanordnung an dem proximalen Ende des Gehäuses abnehmbar befestigt ist; wobei das Gehäuse eine proximal gerichtete Oberfläche an dem proximalen Ende des Gehäuses umfasst; wobei die proximal gerichtete Oberfläche von einer ersten Seite zu einer zweiten Seite relativ zu einer Ebene senkrecht zu der Längsachse geneigt ist;
wobei der Verbinder an die proximal gerichtete Oberfläche angrenzt; und
wobei der Verbinder an der röhrenförmigen Außenhülle an einem Punkt neben dem ersten Ende der proximal gerichteten Oberfläche schwenkbar befestigt ist.

## Revendications

1. Ensemble capuchon d'un dispositif d'administration de médicament, l'ensemble capuchon comprenant :
une enveloppe externe tubulaire (1), un extracteur de protection (2) et un connecteur (3) ;
dans lequel l'enveloppe externe tubulaire (1) s'étend le long d'un axe longitudinal (L) entre une extrémité proximale (11) et une extrémité distale (12) ;
dans lequel l'extracteur de protection (2) est au moins partiellement agencé à l'intérieur de l'enveloppe externe tubulaire (1) ; dans lequel l'extracteur de protection (2) est mobile par rapport à l'enveloppe externe tubulaire (1) dans la direction de l'axe longitudinal (L) ; et
dans lequel le connecteur (3) est agencé à l'intérieur de l'enveloppe externe tubulaire (1) ;
dans lequel le connecteur (3) est relié de manière mobile à la fois à l'enveloppe externe tubulaire (1) et à l'extracteur de protection (2), dans lequel le connecteur (3) est conçu pour être déplacé dans la direction proximale par un mouvement de l'enveloppe externe tubulaire dans la direction proximale, de telle sorte que l'extracteur de protection (2) est déplacé sur une distance inférieure à une distance parcourue par l'enveloppe externe tubulaire (1) ;
dans lequel le connecteur (3) est fixé de manière rotative à l'enveloppe externe tubulaire (1) ;
et **caractérisé en ce que**
le connecteur (3) est fixé de manière pivotante à l'enveloppe externe tubulaire ;
et dans lequel le connecteur comprend un corps de connecteur ; et dans lequel le corps de connecteur comprend un anneau ou un anneau de retenue.

2. Ensemble bouchon selon la revendication 1, dans lequel le connecteur (3) est fixé de manière rotative à l'enveloppe externe tubulaire (1) ou à l'extracteur de protection (2), de sorte qu'un déplacement de l'enveloppe externe tubulaire (1) par rapport à l'extracteur de protection (2) dans la direction de l'axe longitudinal (L) entraîne la rotation du connecteur (3) par rapport à l'enveloppe externe tubulaire (1).

3. Ensemble capuchon selon la revendication 1 ou 2, dans lequel l'ensemble capuchon comprend une extension axiale s'étendant dans la direction de l'axe longitudinal ; et dans lequel l'extension axiale est agencée entre l'extracteur de protection et le corps de connecteur.

4. Ensemble capuchon selon la revendication 3, dans lequel le corps de connecteur comprend l'extension axiale s'étendant dans la direction de l'axe longitudinal du corps de connecteur vers l'extracteur de protection.

5. Ensemble capuchon selon la revendication 4, dans lequel l'extension axiale du corps de connecteur est adjacente à une surface orientée de manière distale de l'extracteur de protection.

6. Ensemble capuchon selon l'une quelconque des revendications précédentes, dans lequel le corps de connecteur comprend une extension transversale s'étendant à partir du corps de connecteur dans une direction transversale à l'axe longitudinal ; et dans lequel l'extension transversale est fixée de manière pivotante à l'enveloppe externe tubulaire.

7. Ensemble capuchon selon la revendication 6, dans lequel une découpe ou un renfoncement est agencé dans une paroi de l'enveloppe externe tubulaire ; et dans lequel la saillie transversale du connecteur est positionnée dans la découpe ou le renfoncement.

8. Ensemble bouchon selon l'une quelconque des revendications précédentes, dans lequel l'extracteur de protection comprend un corps interne, le corps interne comprenant une pince destinée à mettre en prise une protection de l'élément d'administration du dispositif d'administration de médicament ; et dans lequel la pince s'étend à partir d'une paroi du corps interne.

9. Ensemble bouchon selon la revendication 8, dans lequel le corps interne s'étend le long de l'axe longitudinal entre une extrémité proximale et une extrémité distale ; et dans lequel la pince est agencée plus près de l'extrémité distale du corps interne que de l'extrémité proximale du corps interne.

10. Ensemble capuchon selon l'une quelconque des revendications précédentes, dans lequel l'extracteur de protection est fixé de manière mobile à l'enveloppe externe tubulaire de l'ensemble capuchon par une mise en prise par encliquetage.

11. Ensemble capuchon selon la revendication 10, dans lequel la mise en prise par encliquetage est formé par une rainure d'encliquetage et un bras d'encliquetage ; et dans lequel le bras d'encliquetage est mobile à l'intérieur de la rainure d'encliquetage sur une distance prédéterminée.

12. Sous-ensemble d'un dispositif d'administration de médicament, le sous-ensemble comprenant l'ensemble capuchon selon l'une quelconque des revendications 3 à 11, dans lequel le sous-ensemble comprend un boîtier destiné à recevoir un contenant de médicament du dispositif d'administration de médicament ; dans lequel le boîtier s'étend le long de l'axe longitudinal entre une extrémité proximale et une extrémité distale ; dans lequel l'ensemble capuchon est fixé de manière amovible à l'extrémité proximale du boîtier ; dans lequel le boîtier comprend une surface orientée de manière proximale au niveau de l'extrémité proximale du boîtier ; dans lequel la surface orientée de manière proximale est inclinée d'un premier côté vers un second côté par rapport à un plan perpendiculaire à l'axe longitudinal ;
dans lequel le connecteur est adjacent à la surface orientée de manière proximale ; et
dans lequel le connecteur est fixé de manière pivotante à l'enveloppe externe tubulaire au niveau d'un point le plus proche de la première extrémité de la surface orientée de manière proximale.
